# EUROPEAN PATENT SPECIFICATION

(11) **EP 1 234 562 B1**
(45) Date of publication and mention of the grant of the patent: **16.03.2005**
(21) Application number: 02251285.9
(22) Date of filing: 25.02.2002
(51) Int. Cl.: A61F 13/472, A61F 13/15

(54) **Sanitary absorbent article**
Damenbinde
Serviette hygiénique

(30) Priority: 23.02.2001 JP 2001048479; 05.06.2001 JP 2001169004
(43) Date of publication of application: 28.08.2002
(73) Proprietor: Uni-Charm Corporation, Shikokuchuo-shi, Ehime-ken (JP)
(72) Inventor: Mizutani, Satoshi, c/o Technical Center, Mitoyo-gun, Kagawa-ken 769-1602 (JP); Yamaki, Koichi, c/o Technical Center, Mitoyo-gun, Kagawa-ken 769-1602 (JP); Noda, Yuki, c/o Technical Center, Mitoyo-gun, Kagawa-ken 769-1602 (JP)
(74) Representative: Parry, Christopher Stephen

(56) References cited:
- EP-A- 0 983 759
- EP-A- 0 985 396
- WO-A-00/19956
- US-A- 5 954 705

## Description

### Field of the Invention

The present invention relates to a hygienic absorbent article for absorbing the menstrual blood and, more particularly, to a hygienic absorbent article which can be held in close contact with the wearer's body so as not to go out of position by the motion of the body.

### Description of Related Art

Typically, hygienic absorbent articles such as sanitary napkins are constructed to have a main body including: a liquid-impermeable back layer (back sheet); a liquid-permeable surface layer (top sheet); and an absorbent layer (absorbent core) sandwiched between the back layer and the surface layer. In addition, a pressure-sensitive adhesive layer is provided on an outer face (garment-facing face) of the back layer. By adhering the pressure-sensitive adhesive layer to an undergarment, the sanitary napkin can be prevented from going out of position with respect to the undergarment.

In order to improve close contact with the private part of a wearer, some of the sanitary napkins are provided at the center of the absorbing region with a protrusion for coming into close contact with the private part.

Conventional sanitary napkins of this type are disclosed, for example, in Japanese Unexamined Utility Model Publication No. 33721/1993 and Japanese Unexamined Patent Publication No. 504486/1998. In both of these disclosures, a protrusion having an absorption power is fixed on the center of the liquid-receiving face of the sanitary napkin. By bringing the protrusion into close contact with the wearer's private part, the ability to trap the menstrual blood is enhanced.

In these sanitary napkins of the prior art, however, the protrusion is fixed without any degree of freedom. Therefore, when the outer face of the back layer is adhered to the inner face of the undergarment through the pressure-sensitive adhesive layer, if the undergarment goes out of position from the private part to cause dislocation of the sanitary napkin, the protrusion easily leaves the private part.

Especially when an undergarment having a weak fastening force to the wearer's body is worn and when the sanitary napkin is fixed on the undergarment by the pressure-sensitive adhesive layer, the protrusion easily leaves the crotch of a wearer together with the undergarment while the wearer is walking or in bed, thereby to deteriorate the close contact between the protrusion and the private part.

Moreover, the protrusion provided in the sanitary napkin of the prior art mainly comprises a hydrophilic fibrous layer. In a wet condition, therefore, it is difficult for the protrusion to elastically restore to its original shape. That is, when the protrusion having absorbed a liquid is pressed against the wearer's body, it easily shrinks. As a result, the protrusion cannot exhibit sufficient elastic restoring force to closely contact with the private part but easily leaves the private part, when the sanitary napkin goes out of position together with the undergarment. In addition, the protrusion thus wetted and pressed against the wearer's body easily becomes stiff, thereby giving uncomfortable feeling to the wearer.

Moreover, since the sanitary napkin of the prior art is fixed on the undergarment through the pressure-sensitive adhesive layer provided on the back layer, it moves together with the undergarment to cause dislocation from the private part. Therefore, it is required to interpose the absorbent layer and the back layer over a wide area between the private part and the undergarment. As a result, the entire size of the sanitary napkin becomes relatively large.

### SUMMARY OF THE INVENTION

The present invention has been worked out in view of the foregoing shortcomings of the prior art and has an object to provide a hygienic absorbent article, a portion of which to contact with the private part of a wearer is movable with respect to a main body of the article so as to keep close contact with the private part.

According to a first aspect of the invention, there is provided a hygienic absorbent article comprising:
a main body including a back layer and an absorbent layer having two side edges extending in a longitudinal direction of the article and laid on the back layer;
a three-dimensional portion extending in the longitudinal direction of the article and provided over the absorbent layer, the three-dimensional portion being fixed at two fixed ends thereof onto the main body, the fixed ends being located between the two side edges of the absorbent layer, the three-dimensional portion including two connecting portions to rise from the fixed ends and a skin-contacting portion extending between upper ends of the connecting portions to have a pair of free ends, each free end being located closer to corresponding one of the side edges of the absorbent layer than the fixed end, at least an upper face of the skin-contacting portion being made of a liquid-permeable material; and
elastic members provided in the three-dimensional portion to extend in the longitudinal direction of the article for applying a longitudinal elastic contractive force to the three-dimensional portion so that the three-dimensional portion is allowed to rise to have the free ends of the skin-contacting portion spaced above the main body while being anchored at the fixed ends to the main body.

Here, the three-dimensional portion may be formed by folding a single sheet to extend from one fixed end and come back to the other fixed end.

According to a second aspect of the invention, there is provided a hygienic absorbent article comprising:
a main body including a back layer and an absorbent layer having two side edges extending in a longitudinal direction of the article and laid on the back layer;
first and second three-dimensional portions extending in the longitudinal direction of the article and symmetrically provided over the absorbent layer with respect to a longitudinally extending centerline of the absorbent layer, the first and second three-dimensional portions being fixed at fixed ends thereof onto the main body, the fixed ends being located between the two side edges of the absorbent layer, each three-dimensional portion including a connecting portion to rise from the fixed end and a skin-contacting portion bent from the connecting portion outwardly to have a free end located closer to corresponding one of the side edges of the absorbent layer than the fixed end, at least an upper face of the skin-contacting portion being made of a liquid-permeable material; and
elastic members provided in the first and second three-dimensional portions to extend in the longitudinal direction of the article for applying a longitudinal elastic contractive force to the first and second three-dimensional portion so that the first and second three-dimensional portions are allowed to rise to have the free ends of the skin-contacting portions spaced above the main body while being anchored at the fixed ends to the main body.

In the hygienic absorbent article according to the second aspect of the invention, the first three-dimensional portion may be formed from a first sheet extending over the absorbent layer from one side edge of the absorbent layer; and the second three-dimensional portion may be formed from a second sheet extending over the absorbent layer from the other side edge of the absorbent layer. The connecting portion of the first three-dimensional portion and the connecting portion of the second three-dimensional portion may be joined at least partiallyPreferably, the fixed end of the first three-dimensional portion and the fixed end of the second three-dimensional portion are spaced apart in a direction perpendicular to the longitudinal direction by no more than 1/2 of the distance between the two side edges of the absorbent layer. Alternatively, the first and second three-dimensional portions may be formed from a single liquid-permeable sheet to have a common fixed end.

Here, a hydrophilic material layer may further be provided inside the three-dimensional portion(s). For example, in the first aspect of the invention, a hydrophilic material layer may be provided in at least one of the connecting portion and the skin-contacting portion. In the second aspect of the invention, on the other hand, a hydrophilic material layer may be provided in at least one of the connecting portion and the skin-contacting portion of each three-dimensional portion, or it may be provided between the connecting portion of the first three-dimensional portion and the connecting portion of the second three-dimensional portion.

In both the first and second aspects of the invention, a pressure-sensitive adhesive layer may be provided on an outer face of the back layer for preventing dislocation of the article during use.

Alternatively, it is possible to fit the skin-contacting portion(s) between the labia of a female genital organ when the hygienic absorbent article is worn. In this case, it is preferred that no pressure-sensitive adhesive for preventing dislocation of the article during use is provided on an outer face of the back layer, but the hygienic absorbent article is allowed to be retained on the body of a wearer by closing force of the labia. In order to make the skin-contacting portion(s) fit between the labia, preferably, the three-dimensional portion(s) may have a length of 50 to 70 mm in the longitudinal direction of the article and a width of 20 to 40 mm between the free ends thereof in a direction perpendicular to the longitudinal direction. Moreover, it is preferred that the main body has a length of 80 to 120 mm in the longitudinal direction of the article and a width of 40 to 60 mm in a direction perpendicular to the longitudinal direction.

Here, the main body may further comprise a liquid-permeable surface layer covering the absorbent layer, and the three-dimensional portion(s) may be fixed at the fixed ends thereof on the surface layer.

### BRIEF DESCRIPTION OF THE DRAWINGS

The present invention will be understood more fully from the detailed description given hereinafter and from the accompanying drawings of the preferred embodiments of the present invention, which, however, should not be taken to be limitative to the invention, but are for explanation and understanding only.

In the drawings:
Fig. 1 is a perspective view showing a hygienic absorbent article according to a first embodiment of the invention;
Fig. 2 is a sectional view taken along line II - II of Fig. 1;
Fig. 3 is a sectional view taken along line III - III of Fig. 1;
Fig. 4 is a sectional view corresponding to Fig. 2 but shows a modification of the first embodiment;
Fig. 5 is a sectional view corresponding to Fig. 2 but shows a modification of the first embodiment;
Fig. 6 is a perspective view showing a second embodiment of the invention;
Fig. 7 is a sectional view taken along line VII - VII of Fig. 6;
Fig. 8 is a sectional view taken along line VIII - VIII of Fig. 6;
Fig. 9 is a sectional view corresponding to Fig. 7 but shows a modification of the second embodiment;
Fig. 10 is a sectional view corresponding to Fig. 7 but shows a modification of the second embodiment; and
Fig. 11 is a sectional view corresponding to Fig. 7 but shows a hygienic absorbent article according to a third embodiment of the invention.

### DESCRIPTION OF THE PREFERRED EMBODIMENTS

The present invention will be discussed hereinafter in detail in terms of the preferred embodiments according to the present invention with reference to the accompanying drawings. In the following description, numerous specific details are set forth in order to provide a thorough understanding of the present invention. It will be obvious, however, to those skilled in the art that the present invention may be practiced without these specific details. In other instance, well-known structure are not shown in detail in order to avoid unnecessary obscurity of the present invention.

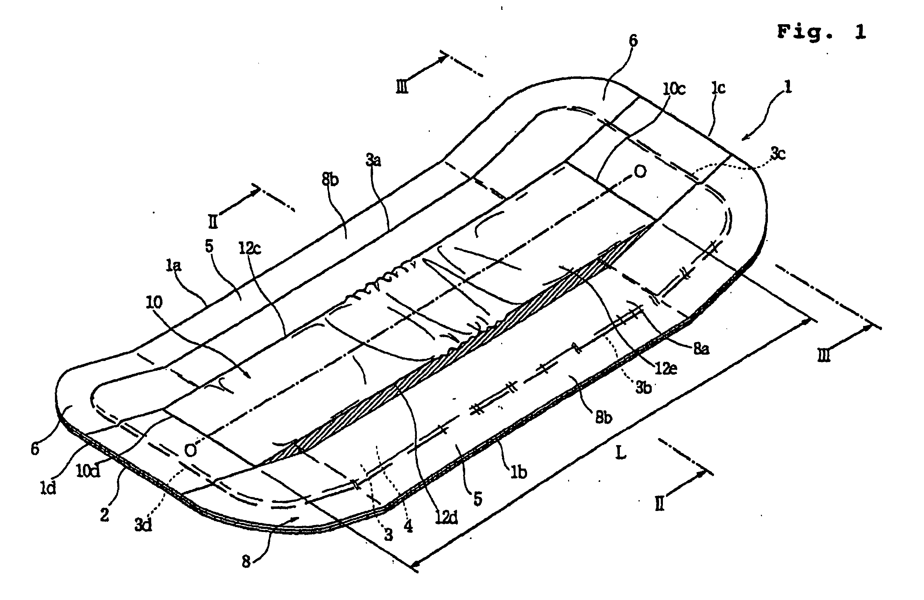

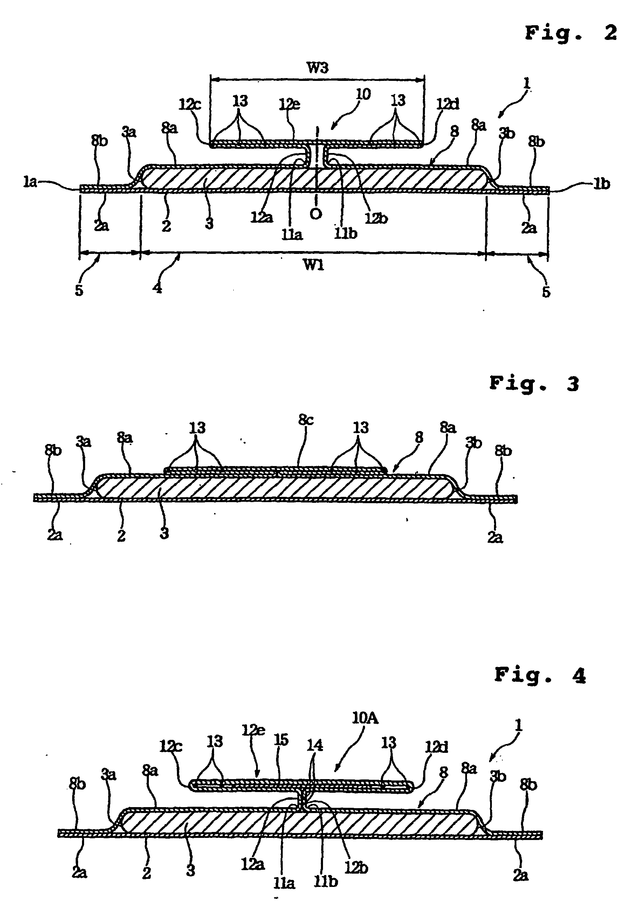

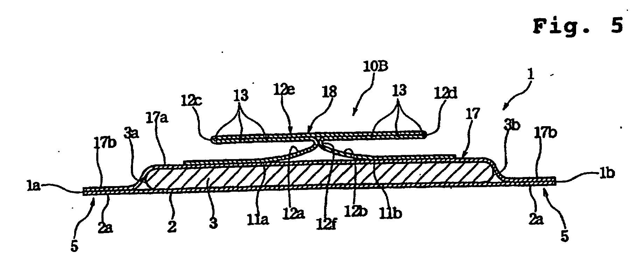

Fig. 1 is a perspective view showing a hygienic absorbent article 1, as taken from a liquid receiving side, according to a first embodiment of the invention; Fig. 2 is a sectional view taken along line II - II of Fig. 1; and Fig. 3 is a sectional view taken along line III - III of Fig. 1. Moreover, Figs. 4 and 5 are sectional views corresponding to Fig. 2 but show modifications of the first embodiment.

The hygienic absorbent article 1, as shown in Figs. 1 to 3, has two longitudinally extending side edges 1a and 1b, and a front edge 1c and a rear edge 1d. A liquid-impermeable back layer 2 to confront an external wear such as an undergarment is sized equally to the entirety of the hygienic absorbent article 1. Over the back layer 2, there is laid an absorbent layer 3. This absorbent layer 3 has two longitudinally extending side edges 3a and 3b, and a front edge 3c and a rear edge 3d, but is sized just smaller than the back layer 2. In the hygienic absorbent article 1, the area having the absorbent layer 3 is called a liquid absorbing region 4.

As shown in a transverse section of Fig. 2, between the side edges 3a and the side edge 1a and between the side edges 3b and the side edge 1b, there are formed side flaps 5 and 5, respectively. On the other hand, between the front edge 3c and the front edge 1c and between the rear edge 3d and the rear edge 1d, there are formed front and rear flaps 6 and 6, respectively.

The hygienic absorbent article 1 is provided on its liquid receiving side with a liquid-permeable sheet 8. Over the liquid absorbing region 4 having the absorbent layer 3, as shown in Fig. 1, there is provided a three-dimensional portion 10 formed from the liquid-permeable sheet 8 and extending over a region indicated by a longitudinal length L. In the region L, as shown in Fig. 2, the three-dimensional portion 10 is formed from a portion of the liquid-permeable sheet 8 extending between 11a and 11b, wherein the numeral 11a indicates a first fixed end of the three-dimensional portion 10 which is spaced toward the center of the absorbent layer 3 from the side edge 3a by a predetermined distance; and the numeral 11b indicates a second fixed end of the three-dimensional portion 10 which is spaced toward the center of the absorbent layer 3 from the side edge 3b by a predetermined distance.

The first fixed end 11a and the second fixed end 11b are arranged at symmetric positions with respect to a centerline O - O extending longitudinally of the hygienic absorbent article 1. The first fixed end 11a and the second fixed end 11b are formed such that the liquid-permeable sheet 8 and the upper face of the absorbent layer 3 are fixed together (e.g., bonded with a hot-melt adhesive or thermally fused) on a continuous line or an intermittent line parallel to the centerline O - O.

As shown in Fig. 2, the liquid-permeable sheet 8 also provides a surface layer 8a covering the surface of the absorbent layer 3 at its portion extending from the side edge 3a of the absorbent layer 3 to the first fixed end 11a and at its portion extending from the side edge 3b of the absorbent layer 3 to the second fixed end 11b. Moreover, the liquid-permeable sheet 8 has extensions 8b and 8b extending beyond the side edges 3a and 3b of the absorbent layer 3, respectively. Here, the back layer 2 also has extensions 2a and 2a extending beyond the side edges 3a and 3b of the absorbent layer 3, respectively. At the side flaps 5 and 5, the extensions 2a and 2a of the back layer 2 and the extensions 8b and 8b of the liquid-permeable sheet 8 are bonded with a hot-melt type adhesive or the like.

At the front and rear flaps 6 and 6, too, extensions of the back layer 2 extending beyond the front edge 3c and the rear edge 3d of the absorbent layer 3 and extensions of the liquid-permeable sheet 8 extending beyond the front edge 3c and the rear edge 3d of the absorbent layer 3 are bonded with a hot-melt type adhesive or the like.

The three-dimensional portion 10 thus provided in the region L is of symmetrical shape with respect to the centerline O - O, as shown in the sectional view of Fig. 2. In the three-dimensional portion 10, the liquid-permeable sheet 8 extends upwardly from the first and second fixed ends 11a and 11b to form connecting portions (rising walls) 12a and 12b, respectively. Between the upper ends of the connecting portions 12a and 12b, the liquid-permeable sheet 8 extends symmetrically with respect to the centerline O - O.

Thus, the three-dimensional portion 10 has a T-shaped section, the head of which has left and right free ends 12c and 12d and is called band-shaped, skin-contacting portion 12e. The free end 12c is located closer to the side edge 3a than the first fixed end 11a; and the free end 12d is located closer to the side edge 3b than the second fixed end 11b. Since the upper face of the skin-contacting portion 12e is formed from a portion of the liquid-permeable sheet 8 extending between the free ends 12c and 12d, it is permeable to liquid.

As shown in Fig. 1, the three-dimensional portion 10 has a front end 10c and a rear end 10d. Between the front end 10c of the three-dimensional portion 10 and the front edge 1c of the hygienic absorbent article 1 and between the rear end 10d of the three-dimensional portion 10 and the rear edge 1d of the hygienic absorbent article 1, as shown in Fig. 3, the liquid-permeable sheet 8 provides flat, folded portions 8c. In the liquid absorbing region 4, the folded portions 8c are entirely fixed to the surface of the absorbent layer 3. In the front and rear flaps 6 and 6, the folded portions 8c are entirely fixed to the upper face of the back layer 2.

In the three-dimensional portion 10 and the folded portion 8c, as shown in Figs. 2 and 3, there are provided a plurality of elastic members 13 in the form of filaments or strips. These elastic members 13 are disposed between and fixed to the confronting faces of the folded liquid-permeable sheet 8. In the three-dimensional portion 10 shown in Fig. 2, the elastic members 13 are disposed at least in the skin-contacting portion 12e to extend in parallel with the free ends 12c and 12d of the skin-contacting portion 12e.

The elastic members 13 are fixed to the liquid-permeable sheet 8 while being stretched to a predetermined length. Therefore, in a free state where no external force is applied to the hygienic absorbent article 1, the three-dimensional portion 10 is elastically contracted by elastic contractive force of the elastic members 13 in such a direction that the front end 10c and the rear end 10d may approach each other. As a result, the hygienic absorbent article 1 is so curved and deformed that the back layer 2 may bulge outward. As a result of this curved/deformed state, the free ends 12c and 12d of the skin-contacting portion 12e of the three-dimensional portion 10 leave the surface layer 8a so that the three-dimensional portion 10 rises into a T-shaped state in the region L.

The hygienic absorbent article 1 may further be provided on the outer face (or the lower face in the drawings) of the back layer 2 with a pressure-sensitive adhesive layer (not shown). With this pressure-sensitive adhesive layer, the hygienic absorbent article 1 can be fixed on the inner face of the external wear such as undergarment, like conventional sanitary napkins. By fixing the back layer 2 on the inner face of the undergarment through the pressure-sensitive adhesive layer and by putting this undergarment on a wearer's body, the liquid-receiving side of the hygienic absorbent article 1 can be applied to the private part of the wearer.

In the worn state, the skin-contacting portion 12e of the three-dimensional portion 10 comes into close contact with the wearer's private part, and then, the absorbent layer 3 is pushed onto the wearer's body by the undergarment, so that the connecting portions 12a and 12b are flexed to bring the skin-contacting portion 12e into substantial contact with the surface layer 8a. Here, since the skin-contacting portion 12e of the three-dimensional portion 10 is always biased away from the surface layer 8a by the elastic force of the elastic members 13, the skin-contacting portion 12e can be always kept in close contact with the wearer's private part.

Therefore, even if an undergarment having a poor fastening force is worn and the back layer 2 fixed on the undergarment through the pressure-sensitive adhesive layer laterally goes out of position together with the undergarment, the skin-contacting portion 12e is always enabled to keep abutment against the private part by free deformation of the connecting portions 12a and 12b.

Accordingly, a menstrual blood discharged from the wearer's private part can always be received by the skin-contacting portion 12e of the three-dimensional portion 10. In case where no displacement of the undergarment occurs, the menstrual blood thus received permeates through the skin-contacting portion 12e and further through the surface layer 8a and is then absorbed by the absorbent layer 3. In case where the back layer 2 moves laterally together with the undergarment as described above so that the absorbent layer 3 goes laterally out of position, on the other hand, the menstrual blood permeates through the skin-contacting portion 12e, through the connecting portions 12a and 12b, and further through the surface layer 8a and can reach the absorbent layer 3. Therefore, the menstrual blood hardly leaks laterally of the hygienic absorbent article 1.

Here, after absorption of the menstrual blood, the absorbent layer 3 becomes stiff. However, since the skin-contacting portion 12e of the three-dimensional portion 10 is in close contact with the wearer's private part, the stiffness of the wetted absorbent layer 3 is not directly felt by the private part, preventing deterioration of wearing feel.

Modifications shown in Figs. 4 and 5 are identical in their entire basic structures to that of the hygienic absorbent article 1 shown in Figs. 1 to 3 but are different in their structures of the three-dimensional portion formed in the region L from that of the three-dimensional portion 10 shown in Fig. 2.

In a three-dimensional portion 10A shown in Fig. 4, the connecting portions 12a and 12b rising from the first fixed end 11a and the second fixed end 11b are joined to each other, and a plurality of elastic members 14 are disposed between the joined connecting portions 12a and 12b, in addition to the elastic members 13 disposed in the skin-contacting portion 12e. This hygienic absorbent article is curved by the elastic contractive forces of both the elastic members 13 and the elastic members 14 so that the three-dimensional portion 10A rises in the T-shaped state in the region L. In the skin-contacting portion 12e, moreover, a hydrophilic material layer 15 is disposed between the confronting faces of the folded liquid-permeable sheet 8. This hydrophilic material layer 15 has a liquid absorption power and a liquid holding power and is preferred to have a higher liquid holding power per unit area than that of the liquid-permeable sheet 8. The thickness of the hydrophilic material layer 15 may be arbitrarily selected, as long as it has such liquid holding power and can provide a soft feel to the skin. However, it is preferred that the hydrophilic material layer 15 is thinner or softer than the absorbent layer 3 so as to prevent the skin-contacting portion 12e from being stiffened after absorption of the menstrual liquid and to provide a good contact feet to the skin. For example, the hydrophilic material layer 15 is made of tissue paper, air-laid pulp, cotton sheet or the like. Alternatively, a material having an excellent liquid-permeability, such as a sheet made of synthetic resin fibers treated to be hydrophilic, may be provided in place of the hydrophilic material layer 15.

In the modification shown in Fig. 5, over the back layer 2, there is placed the absorbent layer 3, over which a liquid-permeable sheet 17 is laid. This liquid-permeable sheet 17 provides a surface layer 17a covering the absorbent layer 3 at its portion extending between the two side edges 3a and 3b of the absorbent layer 3. Moreover, the liquid-permeable sheet 17 has extensions 17b and 17b extending outwardly beyond the two side edges 3a and 3b, respectively. At the side flaps 5 and 5, the extensions 2a and 2a of the back layer 2 and the extensions 17b and 17b are fixed with a hot-melt type adhesive or the like. Moreover, a liquid-permeable sheet 18 forming a three-dimensional portion 10B is disposed over the surface layer 17a. The liquid-permeable sheet 18 forming the three-dimensional portion 10B has the connecting portions 12a and 12b, which rise from the first fixed end 11a and the second fixed end 11b fixed to the liquid-permeable sheet 17 forming the surface layer 17a. These connecting portions 12a and 12b are joined together at their upper ends to form a join portion 12f. Over this join portion 12f, moreover, there is formed the band-shaped, skin-contacting portion 12e which has the free ends 12c and 12d on the two sides. Here, it is, of course, possible to provide the hydrophilic material layer 15 of Fig. 4 in the skin-contacting portion 12e of Fig. 5, and to provide the elastic members 14 of Fig. 4 between the connecting portions 12a and 12b of Fig. 5.

Although not shown in drawings, moreover, it is also possible to provide a hydrophilic material layer similar to the hydrophilic material layer 15 between the connecting portions 12a and 12b forming the stem of the three-dimensional portions 10, 10A and 10B. With the hydrophilic material layer provided between the connecting portions 12a and 12b, the stem of the three-dimensional portion is allowed to absorb a small amount of menstrual blood. Therefore, the menstrual blood is effectively prevented from leaking laterally of the hygienic absorbent article 1. Here, if the hydrophilic material layer provided between the connecting portions 12a and 12b is thinner or softer than the absorbent layer 3, it hardly obstructs the flexures of the connecting portions 12a and 12b so that the abutment of the three-dimensional portion against the skin can be softened.

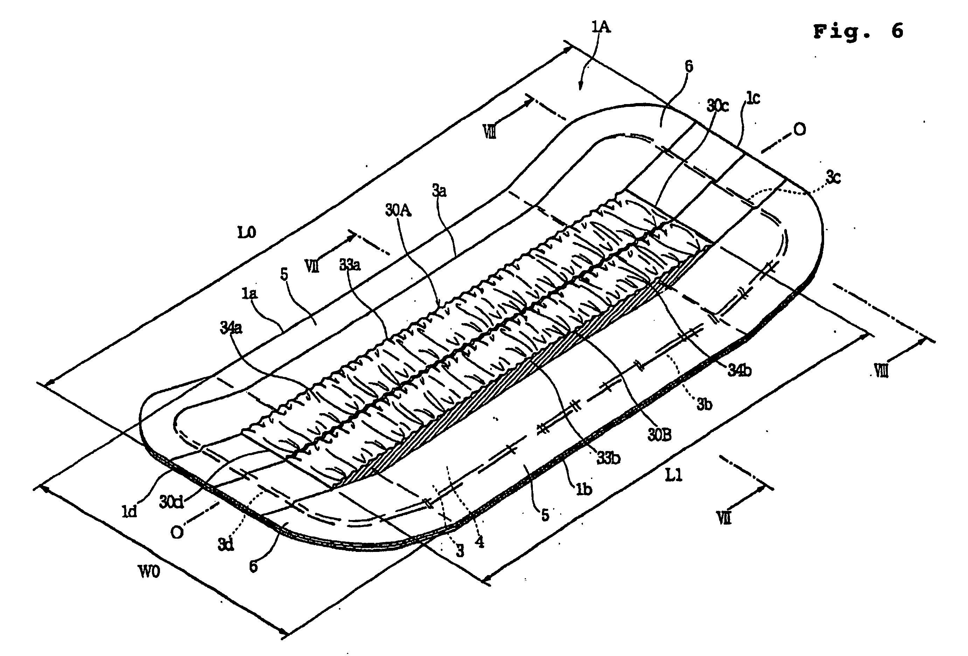

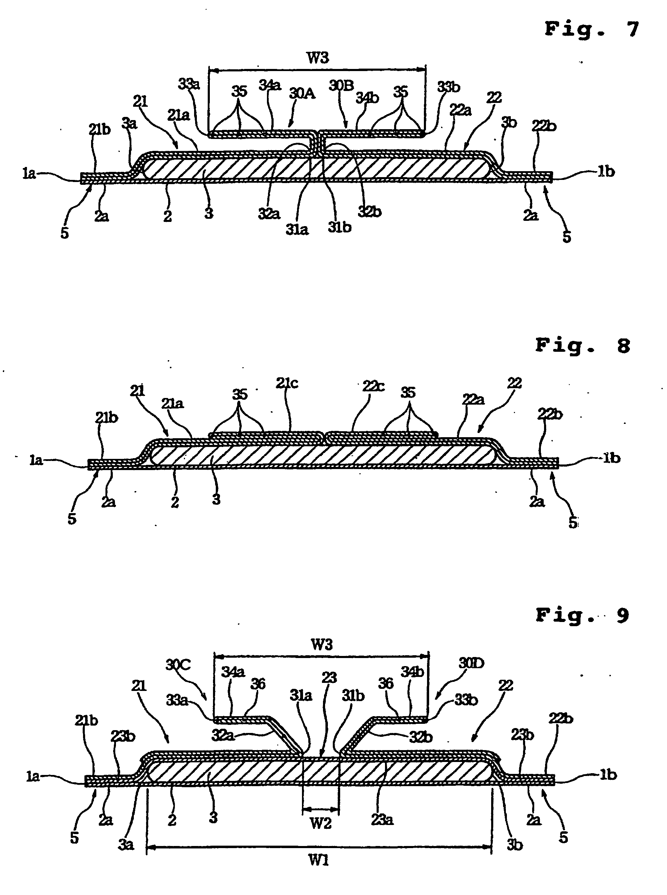

Fig. 6 is a perspective view showing a hygienic absorbent article 1A according to a second embodiment of the invention; Fig. 7 is a sectional view taken along line VII - VII of Fig. 6; Fig. 8 is a sectional view taken along line VIII - VIII of Fig. 6; and Figs. 9 and 10 are sectional views corresponding to the sectional view of Fig. 7 but show modifications of the second embodiment. Here, the detailed description of the portions having the same constructions as those of the first embodiment will be omitted by designating them by the common reference numerals.

In the second embodiment shown in Figs. 6, 7 and 8, the absorbent layer 3 is laid over the back layer 2 having the same size as that shown in Fig. 1. Moreover, symmetrically with respect to the longitudinal centerline O - O, a first liquid-permeable sheet 21 extends from one side edge 1a of the hygienic absorbent article 1A to the vicinity of the centerline O - O, and a second liquid-permeable sheet 22 extends from the other side edge 1b to the vicinity of the centerline O - O.

The first liquid-permeable sheet 21 and the second liquid-permeable sheet 22 are folded in two and are fixed to the surface of the absorbent layer 3 at a first fixed end 31a and at a second fixed end 31b in the vicinity of the centerline O - O. The first and second fixed ends 31a and 31b are formed such that each of the liquid-permeable sheets 21 and 22 and the absorbent layer 3 are fixed together (e.g., bonded with a hot-melt adhesive or thermally fused) on a continuous line or an intermittent line parallel to the centerline O - O.

The first liquid-permeable sheet 21 thus folded in two provides: a surface layer 21a at its portion covering the absorbent layer 3 from the side edge 3a of the absorbent layer 3 to the first fixed end 31a; and an extension 21b at its portion extending outward beyond the side edge 3a of the absorbent layer 3. The extension 2a of the back layer 2 extending outward beyond the side edge 3a and the extension 21b are bonded with a hot-melt type adhesive or the like to form one side flap 5. Similarly, the second liquid-permeable sheet 22 thus folded in two provides: a surface layer 22a at its portion covering the absorbent layer 3 from the side edge 3b of the absorbent layer 3 to the second fixed end 31b; and an extension 22b at its portion extending outward beyond the side edge 3b of the absorbent layer 3. The extension 2a of the back layer 2 extending outward beyond the side edge 3b and the extension 22b are bonded with a hot-melt type adhesive or the like to form the other side flap 5. At the front and rear flaps 6 and 6 shown in Fig. 6, moreover, the first liquid-permeable sheet 21 and the second liquid-permeable sheet 22 are bonded to the back layer 2.

Moreover, the first liquid-permeable sheet 21 provides a first three-dimensional portion 30A at a portion rising from the first fixed end 31a; and the second liquid-permeable sheet 22 provides a second three-dimensional portion 30B at a portion rising from the second fixed end 31b. These first three-dimensional portion 30A and second three-dimensional portion 30B are made symmetric with respect to the centerline O - O to have connecting portions (rising walls) 32a and 32b rising from the first fixed end 31a and the second fixed end 31b, respectively, and skin-contacting portions 34a and 34b bent to the left and right from the upper ends of the connecting portions 32a and 32b, respectively. The connecting portions 32a and 32b are bonded to each other. The skin-contacting portion 34a has a free end 33a located closer to the side edge 3a than the first fixed end 31a; and the skin-contacting portion 34b has a free end 33b located closer to the side edge 3b than the second fixed end 31b.

As shown in Fig. 6, the first and second three-dimensional portions 30A and 30B have front and rear ends 30c and 30d. At the front and rear end portions (as located outside of the front and rear ends 30c and 30d) of the hygienic absorbent article 1A, as shown in Fig. 8, the first liquid-permeable sheet 21 and the second liquid-permeable sheet 22 provide flat, folded portions 21c and 22c, respectively. These folded portions 21c and 22c are folded back in opposite directions with respect to the centerline O - O, and are fixed as a whole onto the absorbent layer 3 or the back layer 2 with an adhesive.

The first liquid-permeable sheet 21 and the second liquid-permeable sheet 22 are provided with a plurality of elastic members 35 extending longitudinally of the article 1A. Therefore, the front ends 30c and the rear ends 30d of the first three-dimensional portion 30A and the second three-dimensional portion 30B are pulled to each other by the elastic contractive force of the elastic members 35 to curve the hygienic absorbent article 1A. As a result, the first three-dimensional portion 30A and the second three-dimensional portion 30B are raised into a T-shaped state away from the surface layers 21a and 22a.

As in the first embodiment, a pressure-sensitive adhesive layer (not shown) may also be provided on the back layer 2 of the hygienic absorbent article 1A. When the hygienic absorbent article 1A is worn by fixing the pressure-sensitive adhesive layer of the back layer 2 on an undergarment, the first three-dimensional portion 30A and the second three-dimensional portion 30B according to the second embodiment function substantially like the three-dimensional portions 10, 10A and 10B shown in the sections of Figs. 2, 4 and 5. As a result, the skin-contacting portions 34a and 34b can contact closely with the wearer's private part so that their close contact with the private part can be easily kept even if the back layer 2 moves.

The skin-contacting portions 34a and 34b are formed from the liquid-permeable sheets 21 and 22, so that the menstrual blood given to the skin-contacting portions 34a and 34b permeates into the skin-contacting portions 34a and 34b and through the connecting portions 32a and 32b and is absorbed by the absorbent layer 3.

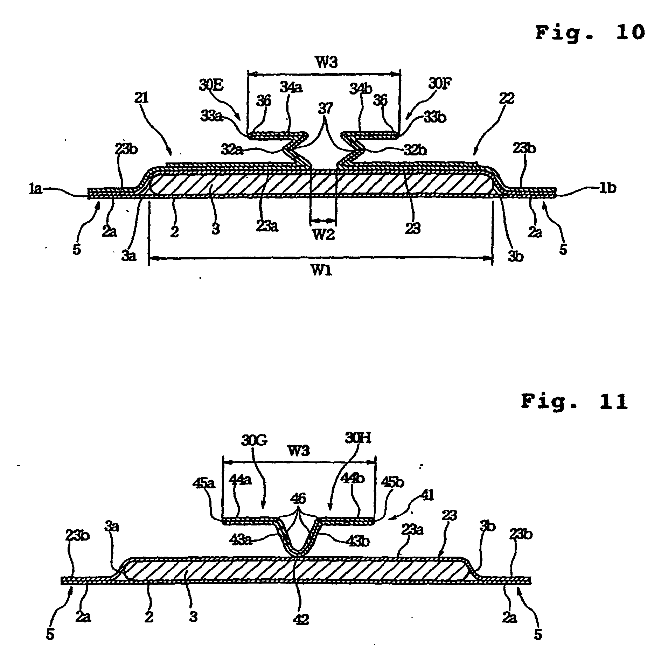

Fig. 9 shows one modification of the second embodiment. In this modification, another liquid-permeable sheet 23 is laid on the absorbent layer 3. This liquid-permeable sheet 23 provides: a surface layer 23a at its portion covering the absorbent layer 3; and extensions 23b and 23b at its portions extending outward beyond the side edges 3a and 3b of the absorbent layer 3. At the side flaps 5 and 5, the extensions 2a and 2a of the back layer 2 and the extensions 23b and 23b are bonded with a hot-melt type adhesive or the like. On the liquid-permeable sheet 23, there are laid the first liquid-permeable sheet 21 and the second liquid-permeable sheet 22 individually in a two-folded state. The first liquid-permeable sheet 21 extends from the side edge 1a of the hygienic absorbent article 1A to over the surface layer 23a thereby to form a first three-dimensional portion 30C rising from the fixed end 31a, at the central portion. On the other hand, the second liquid-permeable sheet 22 extends from the side edge 1b of the hygienic absorbent article 1A to over the surface layer 23a thereby to form a second three-dimensional portion 30D rising from the fixed end 31b, at the central portion. The first three-dimensional portion 30C has the connecting portion 32a and the skin-contacting portion 34a, and the second three-dimensional portion 30D has the connecting portion 32b and the skin-contacting portion 34b. The individual three-dimensional portions 30C and 30D are provided with elastic members 36 extending longitudinally of the article 1A. As shown in Fig. 9, the first fixed end 31a and the second fixed end 31b are formed at symmetric positions with respect to the centerline O - O while being spaced by a distance W2.

Fig. 10 shows another modification of the second embodiment. In the modification shown in Fig. 10, the liquid-permeable sheet 23 is laid on the absorbent layer 3 to form the surface layer 23a covering the absorbent layer 3, as in the modification shown in Fig. 9. Over this surface layer 23a, a first three-dimensional portion 30E is formed from the first liquid-permeable sheet 21, and a second three-dimensional portion 30F is formed from the second liquid-permeable sheet 22. The three-dimensional portions 30E and 30F have the connecting portions 32a and 32b and the skin-contacting portions 34a and 34b, respectively. In the three-dimensional portions 30E and 30F, the skin-contacting portions 34a and 34b are provided with the longitudinally extending elastic members 36, and in addition, the connecting portions 32a and 32b are provided with elastic members 37. In the modification shown in Fig. 10, at the front and rear end portions of the hygienic absorbent article, the first liquid-permeable sheet 21 and the second liquid-permeable sheet 22 are individually folded by crushing Σ shape vertically into a flat state. The individual sheets 21 and 22 thus folded are fixed all over their faces to the surface of the liquid-permeable sheet 23. Thus, the first three-dimensional portion 30E and the second three-dimensional portion 30F can rise to have a Σ-shaped section symmetrically with respect to the centerline O - O when the hygienic absorbent article 1A is curved by the elastic contractive forces of the elastic members 36 and 37.

In the modifications shown in Figs. 9 and 10, when the hygienic absorbent article is worn by fixing the pressure-sensitive adhesive layer of the back layer 2 on an undergarment, the individual skin-contacting portions 34a and 34b contact closely with the wearer' s private part so that the close contact between the skin-contacting portions 34a and 34b and the private part can be kept even when the back layer 2 moves together with the undergarment. Moreover, since the skin-contacting portions 34a and 34b of the separate three-dimensional portions can exhibit the independent behaviors, the skin-contacting portions 34a and 34b can contact closely with the private part independently of each other. Even if the back layer 2 moves laterally or away from the private part, therefore, the skin-contacting portions 34a and 34b and the private part can be kept in the closely contacting state.

In the modification shown in Fig. 10, moreover, since the individual three-dimensional portions 30E and 30F have the Σ shape, they can exhibit high elastic restoring force when compressed (vertically crushed) by the abutting pressure against the skin. Therefore, the three-dimensional portions 30E and 30F of Fig. 10 can further improve the close contact between the skin-contacting portions 34a and 34b and the private part.

Fig. 11 is a sectional view showing a third embodiment having a shape similar to that of the second embodiment of the invention. Here, the detailed description of the portions having the same constructions as those of the first and second embodiments will be omitted by designating them by the common reference numerals.

In the third embodiment shown in Fig. 11, on the back layer 2 having the pressure-sensitive adhesive layer (not shown) on its outer face, there is laid the absorbent layer 3, on which there is laid the liquid-permeable sheet 23. The liquid-permeable sheet 23 provides the surface layer 23a at its portion covering the surface of the absorbent layer 3. Over the liquid-permeable sheet 23, moreover, there is provided a liquid-permeable sheet 41 folded in two.

This liquid-permeable sheet 41 is of a band shape and extends longitudinally of the article. At least in a region L1 of Fig. 6, the longitudinally extending center portion of the band-shaped liquid-permeable sheet 41 is intermittently or continuously fixed (e.g., bonded with an adhesive or thermally fused) on the surface of the liquid-permeable sheet 23 forming the surface layer 23a, thereby forming a fixed end 42. A first three-dimensional portion 30G and a second three-dimensional portion 30H are formed symmetrically with respect to the fixed end 42. The first three-dimensional portion 30G has a connecting portion (rising wall) 43a and a skin-contacting portion 44a; and the second three-dimensional portion 30H has a connecting portion (rising wall) 43b and a skin-contacting portion 44b. In the liquid-permeable sheet 41, moreover, there are fixed a plurality of elastic members 46 extending longitudinally of the article. At the front and rear end portions of the hygienic absorbent article, on the other hand, the liquid-permeable sheet 41 is fixed all over its face to the liquid-permeable sheet 23 forming the surface layer 23a. In a free state, the entire hygienic absorbent article is curved by the elastic contractive force of the elastic members 46, so that the first and second three-dimensional portions 30G and 30H are raised to bring their free ends 45a and 45b away from the liquid-permeable sheet 23.

In this third embodiment, too, the skin-contacting portions 44a and 44b of the first and second three-dimensional portions 30G and 30H can behave independently of each other to contact closely with the wearer's private part in an elastic manner.

In the foregoing individual embodiments and the modifications thereof, a width W3 (although omitted from Figs. 4 and 5) between the free ends of the skin-contacting portion(s) is set within a range of 10 to 60 mm, preferably within a range of 20 to 50 mm, or more preferably within a range of 30 to 50 mm. Moreover, it is preferable that the width W3 is within a range of 20 to 70 % of a width W1 (although omitted from Figs. 4, 5, 7 and 11) between the side edge 3a and the side edge 3b of the absorbent layer 3. With the width between the free ends of the skin-contacting portion(s) being set within the specified ranges, the skin-contacting portion of the three-dimensional portion can easily contact closely with the private part of a wearer. In addition, a distance W2 (although only shown in Figs. 9 and 10) between the fixed ends, where the two connecting portions of the three-dimensional portion(s) start to rise, is preferably 0 to 40 mm, more preferably 0 to 30 mm or most preferably 0 to 20 mm. It is also preferable that the ratio of W2/W1 (i.e., the ratio of the distance W2 between the fixed ends to the width W1 of the absorbent layer 3) is no more than 1/2. With this range, the width W3 between the free ends of the skin-contacting portion(s) can be set within the above-specified preferable range.

On the other hand, a length of the three-dimensional portions, which is indicated at L in Fig. 1 and at L1 in Fig. 6, is preferably within a range of 150 to 400 mm, more preferably within a range of 200 to 360 mm. By setting the length L or L1 within the specified ranges to be sufficiently long in the longitudinal direction of the article, the skin-contacting portion can contact closely with not only the vicinities of the labia of the private part but also the clearance between buttocks, so that the menstrual blood can be suppressed from leaking toward the buttocks while the wearer is in bed.

Moreover, the height of the three-dimensional portions rising from the fixed ends is within a range of 5 to 50 mm, preferably within a range of 10 to 40 mm, or more preferably within a range of 10 to 25 mm.

As has been described hereinabove, the hygienic absorbent articles of the foregoing embodiments and modifications thereof can be used by simply applying the skin-contacting portion(s) to the wearer's private part. However, they may also be used by fitting the skin-contacting portion(s) between the labia of a female genital organ. In detail, the skin-contacting portion of the three-dimensional portion (10, 10A, or 10B) or the skin-contacting portions of the first and second three-dimensional portions (30A and 30B, 30C and 30D, 30E and 30F, or 30G and 30H) may be fitted between the labia so as to be retained by the female genital organ with use of closing force of the labia. As a result, the hygienic absorbent article can be comfortably worn as a whole as if it were integral with the female genital organ. In this case, it is preferable not to provide any pressure-sensitive adhesive layer on the outer face of the back layer 2 so that the back layer 2 may not be fixed on the inner face of the undergarment. The hygienic absorbent article thus worn hardly goes out of position from the female genital organ even if the undergarment goes out of position with respect to the crotch of a wearer. When a menstrual blood is discharged from the female genital organ, therefore, it is always received by the skin-contacting portion. Then, the menstrual blood permeates through the skin-contacting portion, flows down the connecting portion, and thereafter, is absorbed by the absorbent layer 3. In case where the hydrophilic material layer 15 is provided in the skin-contacting portion, as shown in Fig. 4, a small amount of menstrual blood discharged from the organ can be absorbed mainly by this hydrophilic material layer 15. When a large amount of menstrual blood is discharged, on the other hand, most of the menstrual blood not absorbed by the hydrophilic material layer 15 can be absorbed by the underlying absorbent layer 3.

In case where the hygienic absorbent article should be retained by the labia, it is preferred that the hygienic absorbent article has the symmetrical first and second three-dimensional portions in which the skin-contacting portions can behave relatively independently of each other, as shown in Figs. 7, 9, 10 and 11. Especially when the first and second three-dimensional portions are slightly spaced apart from each other so as to enable the skin-contacting portions to behave independently of each other, as shown in Figs. 9, 10 and 11, the skin-contacting portions can be easily retained between the labia.

In the embodiments and modifications shown in Figs. 7, 9, 10 and 11, too, there may be adopted a structure in which a hydrophilic material layer similar to the hydrophilic material layer 15 of Fig. 4 is provided in the skin-contacting portion of each three-dimensional portion. With the hydrophilic material layer being provided in the skin-contacting portion, this skin-contacting portion can be thickened to have a certain degree of stiffness. Therefore, when retained between the labia, the paired skin-contacting portions do not easily come off the labia. Moreover, such a thin, hydrophilic material layer similar to the hydrophilic material layer 15 of Fig. 4 may be provided between the connecting portions 32a and 32b of Fig. 7. It may also be provided in each of the connecting portions 32a and 32b of Figs. 7, 9, and 10 or in each of the connecting portions 43a and 43b of Fig 11 (i.e., between the confronting faces of the folded sheet at the connecting portions 32a and 32b or 43a and 43b).

In case where the hygienic absorbent article is thus designed to be held on the wearer's body by fitting the three-dimensional portion(s) between the labia, its entire size (i.e., the size of the main body) can be made smaller than that of the type to be fixed on an undergarment. Even if the entire size is made smaller, dislocation hardly occurs between the female genital organ and the absorbent layer 3 due to fitting of the three-dimensional portion(s) between the labia. Therefore, lateral leakage of the menstrual blood can be effectively prevented. Moreover, it is unnecessary to make the back layer 2 sufficiently larger than the absorbent layer 3, but it is possible to give a substantially equal size to the back layer 2 and the absorbent layer 3. Alternatively, the left and right side flaps 5 and 5 and the front and rear flaps 6 and 6 can be made as narrow as to have a width of 5 mm or less or 3 mm or less, so that the hygienic absorbent article can be reduced in its entire size.

In order that the skin-contacting portion(s) may be easily retained between the labia, it is preferable that the width W3 between the free ends of the skin-contacting portion(s) is 20 to 40 mm, and that the length L or L1 of the three-dimensional portion in the longitudinal direction are 50 to 70 mm. Moreover, the hygienic absorbent article can be so small-sized in its entirety as to have a length L0 of about 80 to 120 mm and a width W0 of about 40 to 60 mm (see Fig. 6).

Here will be described materials for making the individual components.

The back layer 2 may be formed from a liquid-impermeable sheet, such as a film composed mainly of a resin such as PE, or a laminate of the film with paper or nonwoven fabric. Alternatively, the back layer 2 is preferably formed from a moisture-permeable (breathable) sheet which is prepared by drawing a resin sheet containing an inorganic filler partially or wholly to make it porous.

In the case where the pressure-sensitive adhesive layer to be retained on an external wear such as an undergarment is provided on the outer face of the back layer 2, the pressure-sensitive adhesive layer is preferably elongated in the longitudinal direction of the hygienic absorbent article. In this case, moreover, it is preferable that the pressure-sensitive adhesive layer is covered with a release paper for protecting it before use.

The absorbent layer 3 may be formed from fluff pulp, a laminate of tissue paper, air-laid pulp, an air-laid nonwoven fabric containing fusible fibers, a foamed sheet treated to be hydrophilic, a foamed sheet of cellulose, or a sheet containing superabsorbent polymer. The material for the superabsorbent polymer is exemplified by a granular or fibrous substance selected from a polymer composed mainly of acrylic acid, a graft polymer of starch / acrylic acid, starch, and carboxymethylcellulose.

The absorbent layer 3 is preferred to have a measured value within a range of 5 to 70 mm according to a bending resistance measuring test using the 45-degree cantilever method based on JIS L 1096. Within this range, the absorbent layer 3 can be prevented from being torsionally deformed, and no excessive stiffness is given to the wearer. Here, the confinement of the bending resistance within that range may be supplemented by applying a mechanical pressure to the absorbent layer to have a pressed groove.

Moreover, the absorbent layer 3 is preferred to have a thickness of 1.0 to 5.0 mm.

The liquid-permeable sheet 17 for forming the surface layer 17a shown in Fig. 5 or the liquid-permeable sheet 23 for forming the surface layer 23a shown in Figs. 9 to 11 may be formed from an absorbent material having a wet strength such as air-laid pulp, an apertured plastic film as a liquid-permeable material, a nonwoven fabric composed of hydrophobic synthetic fibers which are treated to be hydrophilic, or an apertured nonwoven fabric.

On the other hand, the liquid-permeable sheet (or the liquid-permeable material) for forming the three-dimensional portions 10, 10A, 10B, 30A and 30B, 30C and 30D, 30E and 30F, and 30G and 30H of the foregoing individual embodiments may be a through air nonwoven fabric having a high bulk and a low liquid residue, or an apertured plastic film having a high liquid shielding effect. For example, the through air nonwoven fabric is made of bicomponent fibers having sheath/core structure, and is preferred to have a basis weight within a range of 20 to 40 g/m² and a thickness within a range of 0.3 to 1.5 mm. On the other hand, the apertured plastic film is preferably made of an olefin resin having a density within a range of 0.90 x 10⁶ to 0.93 x 10⁶ g/cm³ to have a basis weight within a range of 20 to 35 g/m². At this time, the surface of the apertured plastic film is preferred to have an open area ratio within a range of 30 to 70 %. Here, each aperture may be of a round, rhomboid or polygonal shape but should not be limited thereto. Moreover, the area of each aperture or the arrangement pattern of the apertures should not be especially limited. Alternatively, the liquid-permeable sheet (or the liquid-permeable material) for forming the three-dimensional portions may be a point-bonded nonwoven fabric, a spun-laced nonwoven fabric or an air-laid nonwoven fabric. These nonwoven fabrics may contain regenerated cellulose fibers (such as viscose rayon or acetate rayon) or natural cellulose fibers, in addition to the aforementioned bicomponent fibers. A spun-bonded or melt-blown nonwoven fabric may also be employed. The apertured plastic film or the nonwoven fabric may be employed alone or laid on another apertured plastic film or nonwoven fabric having the same structure. Of course, it is possible to laminate different kinds of apertured plastic film or nonwoven fabric.

In the foregoing embodiments and their modifications, each three-dimensional portion is formed from a single liquid-permeable sheet. However, the connecting portion of each three-dimensional portion may be made of a hydrophobic or water-repellent material to be liquid-impermeable, as long as the upper face of the skin-contacting portion of each three-dimensional portion is made of a liquid-permeable material. In this case, the menstrual blood given to the skin-contacting portion closely contacting with the private part of a wearer is guided along the liquid-impermeable connecting portion into the absorbent layer.

Although the present invention has been illustrated and described with respect to exemplary embodiments thereof, it should be understood by those skilled in the art that the foregoing and various other changes, omission and additions may be made therein and thereto, without departing from the spirit and scope of the present invention. Therefore, the present invention should not be understood as limited to the specific embodiments set out above but to include all possible embodiments which can be embodied within a scope encompassed and equivalent thereof with respect to the feature set out in the appended claims.

## Claims

1. A hygienic absorbent article comprising:
a main body including a back layer and an absorbent layer having two side edges extending in a longitudinal direction of the article and laid on the back layer;
a three-dimensional portion extending in the longitudinal direction of the article and provided over the absorbent layer, the three-dimensional portion being fixed at two fixed ends thereof onto the main body, the fixed ends being located between the two side edges of the absorbent layer, the three-dimensional portion including two connecting portions to rise from the fixed ends and a skin-contacting portion extending between upper ends of the connecting portions to have a pair of free ends, each free end being located closer to corresponding one of the side edges of the absorbent layer than the fixed end, at least an upper face of the skin-contacting portion being made of a liquid-permeable material; and
elastic members provided in the three-dimensional portion to extend in the longitudinal direction of the article for applying a longitudinal elastic contractive force to the three-dimensional portion so that the three-dimensional portion is allowed to rise to have the free ends of the skin-contacting portion spaced above the main body while being anchored at the fixed ends to the main body.

2. A hygienic absorbent article as set forth in Claim 1,
wherein the three-dimensional portion is formed by folding a single sheet to extend from one fixed end and come back to the other fixed end.

3. A hygienic absorbent article as set forth in Claim 1,
wherein a hydrophilic material layer is provided in at least one of the connecting portion and the skin-contacting portion.

4. A hygienic absorbent article as set forth in Claim 1,
wherein a pressure-sensitive adhesive layer is provided on an outer face of the back layer for preventing dislocation of the article during use.

5. A hygienic absorbent article as set forth in Claim 1,
wherein the skin-contacting portion is to be fitted between the labia of a female genital organ when the hygienic absorbent article is worn.

6. A hygienic absorbent article as set forth in Claim 5,
which is allowed to be retained on the body of a wearer by closing force of the labia, without providing any pressure-sensitive adhesive for preventing dislocation of the article during use on an outer face of the back layer.

7. A hygienic absorbent article as set forth in Claim 5,
wherein the three-dimensional portion has a length of 50 to 70 mm in the longitudinal direction of the article and a width of 20 to 40 mm between the free ends thereof in a direction perpendicular to the longitudinal direction.

8. A hygienic absorbent article as set forth in Claim 5,
wherein the main body has a length of 80 to 120 mm in the longitudinal direction of the article and a width of 40 to 60 mm in a direction perpendicular to the longitudinal direction.

9. A hygienic absorbent article as set forth in Claim 1,
the main body further comprises a liquid-permeable surface layer covering the absorbent layer, and the three-dimensional portion is fixed at the fixed ends thereof on the surface layer.

10. A hygienic absorbent article comprising:
a main body including a back layer and an absorbent layer having two side edges extending in a longitudinal direction of the article and laid on the back layer;
first and second three-dimensional portions extending in the longitudinal direction of the article and symmetrically provided over the absorbent layer with respect to a longitudinally extending centerline of the absorbent layer, the first and second three-dimensional portions being fixed at fixed ends thereof onto the main body, the fixed ends being located between the two side edges of the absorbent layer, each three-dimensional portion including a connecting portion to rise from the fixed end and a skin-contacting portion bent from the connecting portion outwardly to have a free end located closer to corresponding one of the side edges of the absorbent layer than the fixed end, at least an upper face of the skin-contacting portion being made of a liquid-permeable material; and
elastic members provided in the first and second three-dimensional portions to extend in the longitudinal direction of the article for applying a longitudinal elastic contractive force to the first and second three-dimensional portion so that the first and second three-dimensional portions are allowed to rise to have the free ends of the skin-contacting portions spaced above the main body while being anchored at the fixed ends to the main body.

11. A hygienic absorbent article as set forth in Claim 10,
wherein the first three-dimensional portion is formed from a first sheet extending over the absorbent layer from one side edge of the absorbent layer; and the second three-dimensional portion is formed from a second sheet extending over the absorbent layer from the other side edge of the absorbent layer.

12. A hygienic absorbent article as set forth in Claim 10,
wherein the connecting portion of the first three-dimensional portion and the connecting portion of the second three-dimensional portion are joined at least partially.

13. A hygienic absorbent article as set forth in Claim 10,
wherein the fixed end of the first three-dimensional portion and the fixed end of the second three-dimensional portion are spaced apart in a direction perpendicular to the longitudinal direction by no more than 1/2 of the distance between the two side edges of the absorbent layer.

14. A hygienic absorbent article as set forth in Claim 10,
wherein the first and second three-dimensional portions are formed from a single liquid-permeable sheet to have a common fixed end.

15. A hygienic absorbent article as set forth in Claim 10,
wherein in each of the first and second three-dimensional portions, a hydrophilic material layer is provided in at least one of the connecting portion and the skin-contacting portion.

16. A hygienic absorbent article as set forth in Claim 12,
wherein a hydrophilic material layer is provided between the connecting portion of the first three-dimensional portion and the connecting portion of the second three-dimensional portion.

17. A hygienic absorbent article as set forth in Claim 10,
wherein a pressure-sensitive adhesive layer is provided on an outer face of the back layer for preventing dislocation of the article during use.

18. A hygienic absorbent article as set forth in Claim 10,
wherein the skin-contacting portions are to be fitted between the labia of a female genital organ when the hygienic absorbent article is worn.

19. A hygienic absorbent article as set forth in Claim 18,
which is allowed to be retained on the body of a wearer by closing force of the labia, without providing any pressure-sensitive adhesive for preventing dislocation of the article during use on an outer face of the back layer.

20. A hygienic absorbent article as set forth in Claim 18,
wherein the three-dimensional portions have a length of 50 to 70 mm in the longitudinal direction of the article and a width of 20 to 40 mm between the free ends thereof in a direction perpendicular to the longitudinal direction.

21. A hygienic absorbent article as set forth in Claim 18,
wherein the main body has a length of 80 to 120 mm in the longitudinal direction of the article and a width of 40 to 60 mm in a direction perpendicular to the longitudinal direction.

22. A hygienic absorbent article as set forth in Claim 10,
the main body further comprises a liquid-permeable surface layer covering the absorbent layer, and the three-dimensional portions are fixed at the fixed ends thereof on the surface layer.

## Patentansprüche

1. Hygiene-Absorptionsartikel mit:
einem Basiskörper mit einer hinteren Schicht und einer Absorptionsschicht, die zwei in einer Längsrichtung des Artikels verlaufende und auf die hintere Schicht gelegte Seitenkanten aufweist;
einem dreidimensionalen Abschnitt, der in der Längsrichtung des Artikels verläuft und über der Absorptionsschicht vorgesehen ist, wobei der dreidimensionale Abschnitt an zwei seiner festen Enden an dem Basiskörper befestigt ist, die festen Enden sich zwischen den zwei Seitenkanten der Absorptionsschicht befinden, der dreidimensionale Abschnitt zwei Verbindungsabschnitte, die sich von den festen Enden erheben, und einen Hautkontaktabschnitt aufweist, der zwischen den oberen Enden der Verbindungsabschnitte verläuft, um ein Paar freier Enden zu haben, jedes freie Ende näher an der entsprechenden Seitenkante der Absorptionsschicht liegt als das feste Ende und wenigstens eine obere Seite des Hautkontaktabschnitts aus einem flüssigkeitsdurchlässigen Material ist; und
elastischen Elementen, die in dem dreidimensionalen Abschnitt vorgesehen sind, um in der Längsrichtung des Artikels zum Ausüben einer elastischen Längskontraktionskraft auf den dreidimensionalen Abschnitt verlaufen, so daß der dreidimensionale Abschnitt sich heben kann, um die freien Enden des Hautkontaktabschnitts in Abstand über dem Basiskörper zu haben, während sie an den festen Enden des Basiskörpers verankert werden.

2. Hygiene-Absorptionsartikel nach Anspruch 1, wobei der dreidimensionale Abschnitt durch Faltung einer einzigen Lage gebildet ist, um sich von einem festen Ende aus zurück zu dem anderen festen Ende zu erstrecken.

3. Hygiene-Absorptionsartikel nach Anspruch 1, wobei in dem Verbindungsabschnitt und/oder dem Hautkontaktabschnitt eine hydrophile Materialschicht vorgesehen ist.

4. Hygiene-Absorptionsartikel nach Anspruch 1, wobei eine drucksensitive Klebeschicht an einer Außenseite der hinteren Schicht vorgesehen ist, um ein Verrutschen des Artikels während des Gebrauchs zu verhindern.

5. Hygiene-Absorptionsartikel nach Anspruch 1, wobei der Hautkontaktabschnitt beim Tragen des Hygiene-Absorptionsartikels zwischen den Lippen eines weiblichen Geschlechtsorgans anliegt.

6. Hygiene-Absorptionsartikel nach Anspruch 5, der durch die Schließkraft der Lippen am Körper des Trägers gehalten wird, ohne daß ein drucksensitiver Kleber zum Verhindern des Verrutschens des Artikels während des Gebrauchs auf einer Außenseite der hinteren Schicht aufgebracht wird.

7. Hygiene-Absorptionsartikel nach Anspruch 5, wobei der dreidimensionale Abschnitt eine Länge von 50 bis 70 mm in der Längsrichtung des Artikels und eine Breite von 20 bis 40 mm zwischen seinen freien Enden in einer zur Längsrichtung quer verlaufenden Richtung aufweist.

8. Hygiene-Absorptionsartikel nach Anspruch 5, wobei der Basiskörper eine Länge von 80 bis 120 mm in der Längsrichtung des Artikels und eine Breite von 40 bis 60 mm in einer zur Längsrichtung quer verlaufenden Richtung aufweist.

9. Hygiene-Absorptionsartikel nach Anspruch 1, wobei der Basiskörper ferner eine flüssigkeitsdurchlässige Oberflächenschicht aufweist, die die Absorptionsschicht bedeckt, und der dreidimensionale Abschnitt an seinen festen Enden an der Oberflächenschicht befestigt ist.

10. Hygiene-Absorptionsartikel mit:
einem Basiskörper mit einer hinteren Schicht und einer Absorptionsschicht, die zwei in einer Längsrichtung des Artikels verlaufende und auf die hintere Schicht gelegte Seitenkanten aufweist;
einem ersten und einem zweiten dreidimensionalen Abschnitt, die in der Längsrichtung des Artikels verlaufen und bezüglich einer längs verlaufenden Mittellinie des Absorptionsartikels symmetrisch über der Absorptionsschicht vorgesehen sind, wobei der erste und der zweite dreidimensionale Abschnitt an zwei ihrer festen Enden an dem Basiskörper befestigt sind, die festen Enden sich zwischen den zwei Seitenkanten der Absorptionsschicht befinden, jeder dreidimensionale Abschnitt einen Verbindungsabschnitt, der sich von den festen Enden erhebt, und einen Hautkontaktabschnitt aufweist, der von dem Verbindungsabschnitt nach außen gebogen ist, um ein freies Ende zu haben, das näher an der entsprechenden Seitenkante der Absorptionsschicht liegt als das feste Ende, und wenigstens eine obere Seite des Hautkontaktabschnitts aus einem flüssigkeitsdurchlässigen Material ist; und
elastischen Elementen, die in dem ersten und dem zweiten dreidimensionalen Abschnitt vorgesehen sind, um in der Längsrichtung des Artikels zum Ausüben einer elastischen Längskontraktionskraft auf den ersten und den zweiten dreidimensionalen Abschnitt verlaufen, so daß sich der erste und der zweite dreidimensionale Abschnitt heben können, um die freien Enden des Hautkontaktabschnitts in Abstand über dem Basiskörper zu haben, während sie an den festen Enden des Basiskörpers verankert werden.

11. Hygiene-Absorptionsartikel nach Anspruch 10, wobei der erste dreidimensionale Abschnitt aus einer ersten Lage gebildet ist, die von einer Seitenkante der Absorptionsschicht über die Absorptionsschicht verläuft; und der zweite dreidimensionale Abschnitt aus einer zweiten Lage gebildet ist, die von der anderen Seitenkante der Absorptionsschicht über die Absorptionsschicht verläuft.

12. Hygiene-Absorptionsartikel nach Anspruch 10, wobei der Verbindungsabschnitt des ersten dreidimensionalen Abschnitts und der Verbindungsabschnitt des zweiten dreidimensionalen Abschnitts wenigstens teilweise verbunden sind.

13. Hygiene-Absorptionsartikel nach Anspruch 10, wobei das feste Ende des ersten dreidimensionalen Abschnitts und das feste Ende des zweiten dreidimensionalen Abschnitts in einer zur Längsrichtung quer verlaufenden Richtung einen Abstand voneinander aufweisen, der nicht mehr als die Hälfte des Abstands zwischen den zwei Seitenkanten der Absorptionsschicht beträgt.

14. Hygiene-Absorptionsartikel nach Anspruch 10, wobei der erste und der zweite dreidimensionale Abschnitt aus einer einzigen flüssigkeitsdurchlässigen Lage gebildet sind, um ein gemeinsames festes Ende aufzuweisen.

15. Hygiene-Absorptionsartikel nach Anspruch 10, wobei sowohl in dem ersten als auch in dem zweiten dreidimensionalen Abschnitt eine hydrophile Materialschicht in dem Verbindungsabschnitt und/oder dem Hautkontaktabschnitt vorgesehen ist.

16. Hygiene-Absorptionsartikel nach Anspruch 12, wobei eine hydrophile Materialschicht zwischen dem Verbindungsabschnitt des ersten dreidimensionalen Abschnitts und dem Verbindungsabschnitt des zweiten dreidimensionalen Abschnitts vorgesehen ist.

17. Hygiene-Absorptionsartikel nach Anspruch 10, wobei eine drucksensitive Klebeschicht an einer Außenseite der hinteren Schicht vorgesehen ist, um ein Verrutschen des Artikels während des Gebrauchs zu verhindern.

18. Hygiene-Absorptionsartikel nach Anspruch 10, wobei der Hautkontaktabschnitt beim Tragen des Hygiene-Absorptionsartikels zwischen den Lippen eines weiblichen Geschlechtsorgans anliegt.

19. Hygiene-Absorptionsartikel nach Anspruch 18, der durch die Schließkraft der Lippen am Körper des Trägers gehalten wird, ohne daß ein drucksensitiver Kleber zum Verhindern des Verrutschens des Artikels während des Gebrauchs auf einer Außenseite der hinteren Schicht aufgebracht wird.

20. Hygiene-Absorptionsartikel nach Anspruch 18, wobei die dreidimensionalen Abschnitte eine Länge von 50 bis 70 mm in der Längsrichtung des Artikels und eine Breite von 20 bis 40 mm zwischen ihren freien Enden in einer zur Längsrichtung quer verlaufenden Richtung aufweist.

21. Hygiene-Absorptionsartikel nach Anspruch 18, wobei der Basiskörper eine Länge von 80 bis 120 mm in der Längsrichtung des Artikels und eine Breite von 40 bis 60 mm in einer zur Längsrichtung quer verlaufenden Richtung aufweist.

22. Hygiene-Absorptionsartikel nach Anspruch 10, wobei der Basiskörper ferner eine flüssigkeitsdurchlässige Oberflächenschicht aufweist, die die Absorptionsschicht bedeckt, und die dreidimensionalen Abschnitte an ihren festen Enden an der Oberflächenschicht befestigt sind.

## Revendications

1. Article hygiénique absorbant comprenant:
un corps principal incluant une couche arrière, et une couche absorbante déposée sur la couche arrière et comportant deux bords latéraux s'étendant dans une direction longitudinale de l'article;
une partie tridimensionnelle s'étendant dans la direction longitudinale de l'article et disposée au-dessus de la couche absorbante, la partie tridimensionnelle étant fixée par deux de ses extrémités fixes sur le corps principal, les extrémités fixes étant situées entre les deux bords latéraux de la couche absorbante, la partie tridimensionnelle incluant deux parties de connexion réalisées de manière à s'élever à partir des extrémités fixes ainsi qu'une partie de contact avec la peau s'étendant entre des extrémités supérieures des parties de connexion pour comporter une paire d'extrémités libres, chaque extrémité libre étant située plus près du bord latéral correspondant de la couche absorbante que l'extrémité fixe, au moins une face supérieure de la partie de contact avec la peau étant en une matière perméable aux liquides; et
des éléments élastiques disposés dans la partie tridimensionnelle de manière à s'étendre dans la direction longitudinale de l'article pour appliquer à la partie tridimensionnelle une force de contraction élastique longitudinale afin que la partie tridimensionnelle puisse s'élever pour que les extrémités libres de la partie de contact avec la peau soient espacées au-dessus du corps principal tout en étant ancrées par les extrémités fixes au corps principal.

2. Article hygiénique absorbant selon la revendication 1,
dans lequel la partie tridimensionnelle est formée en pliant une feuille unique pour qu'elle s'étende à partir d'une extrémité fixe et revienne vers l'autre extrémité fixe.

3. Article hygiénique absorbant selon la revendication 1,
dans lequel une couche de matière hydrophile est disposée dans au moins, soit la partie de connexion, soit la partie de contact avec la peau.

4. Article hygiénique absorbant selon la revendication 1,
dans lequel une couche adhésive sensible à la pression est disposée sur une face externe de la couche arrière pour empêcher une dislocation de l'article en cours d'utilisation.

5. Article hygiénique absorbant selon la revendication 1,
dans lequel la partie de contact avec la peau est destinée à être ajustée entre les lèvres d'un organe génital féminin lorsque l'article hygiénique absorbant est porté.

6. Article hygiénique absorbant selon la revendication 5,
qui peut être retenu sur le corps d'une personne porteuse par la force de fermeture des lèvres, sans disposer sur la face externe de la couche arrière aucun adhésif sensible à la pression pour empêcher une dislocation de l'article en cours d'utilisation.

7. Article hygiénique absorbant selon la revendication 5,
dans lequel la longueur de la partie tridimensionnelle est de 50 à 70 mm dans la direction longitudinale de l'article, et sa largeur entre ses extrémités libres est de 20 à 40 mm dans une direction perpendiculaire à la direction longitudinale.

8. Article hygiénique absorbant selon la revendication 5,
dans lequel la longueur du corps principal est de 80 à 120 mm dans la direction longitudinale de l'article et sa largeur est de 40 à 60 mm dans une direction perpendiculaire à la direction longitudinale.

9. Article hygiénique absorbant selon la revendication 1,
dans lequel le corps principal comprend en outre une couche superficielle perméable aux liquides recouvrant la couche absorbante, et la partie tridimensionnelle est fixée par ses extrémités fixes sur la couche superficielle.

10. Article hygiénique absorbant comprenant:
un corps principal incluant une couche arrière, et une couche absorbante déposée sur la couche arrière et comportant deux bords latéraux s'étendant dans une direction longitudinale de l'article;
une première et une deuxième parties tridimensionnelles s'étendant dans la direction longitudinale de l'article et disposées symétriquement au-dessus de la couche absorbante par rapport à un axe s'étendant longitudinalement de la couche absorbante, les première et deuxième parties tridimensionnelles étant fixées par des extrémités fixes de ces dernières sur le corps principal, les extrémités fixes étant situées entre les deux bords latéraux de la couche absorbante, chaque partie tridimensionnelle incluant une partie de connexion réalisée de manière à s'élever à partir de l'extrémité fixe ainsi qu'une partie de contact avec la peau repliée vers l'extérieur à partir de la partie de connexion pour comporter une extrémité libre située plus près du bord latéral correspondant de la couche absorbante que l'extrémité fixe, au moins une face supérieure de la partie de contact avec la peau étant en une matière perméable aux liquides; et
des éléments élastiques disposés dans les première et deuxième parties tridimensionnelles de manière à s'étendre dans la direction longitudinale de l'article pour appliquer aux première et deuxième parties tridimensionnelles une force de contraction élastique longitudinale afin que les première et deuxième parties tridimensionnelles puissent s'élever pour que les extrémités libres des parties de contact avec la peau soient espacées au-dessus du corps principal tout en étant ancrées par les extrémités fixes au corps principal.

11. Article hygiénique absorbant selon la revendication 10,
dans lequel la première partie tridimensionnelle est formée d'une première feuille qui s'étend au-dessus de la couche absorbante à partir d'un premier côté de la couche absorbante; et la deuxième partie tridimensionnelle est formée d'une deuxième feuille qui s'étend au-dessus de la couche absorbante à partir de l'autre côté de la couche absorbante.

12. Article hygiénique absorbant selon la revendication 10,
dans lequel la partie de connexion de la première partie tridimensionnelle et la partie de connexion de la deuxième partie tridimensionnelle sont au moins partiellement assemblées.

13. Article hygiénique absorbant selon la revendication 10,
dans lequel l'extrémité fixe de la première partie tridimensionnelle et l'extrémité fixe de la deuxième partie tridimensionnelle ne sont pas espacées l'une de l'autre, dans une direction perpendiculaire à la direction longitudinale, de plus de la moitié de la distance entre les deux bords latéraux de la couche absorbante.

14. Article hygiénique absorbant selon la revendication 10,
dans lequel les première et deuxième parties tridimensionnelles sont formées à partir d'une unique feuille perméable aux liquides de façon à comporter une extrémité fixe commune.

15. Article hygiénique absorbant selon la revendication 10,
dans lequel une couche de matière hydrophile est disposée, dans chacune des première et deuxième parties tridimensionnelles, dans au moins, soit la partie de connexion, soit la partie de contact avec la peau.

16. Article hygiénique absorbant selon la revendication 12,
dans lequel une couche de matière hydrophile est disposée entre la partie de connexion de la première partie tridimensionnelle et la partie de connexion de la deuxième partie tridimensionnelle.

17. Article hygiénique absorbant selon la revendication 10,
dans lequel une couche d'adhésif sensible à la pression est disposée sur une face externe de la couche arrière pour empêcher une dislocation de l'article en cours de d'utilisation.

18. Article hygiénique absorbant selon la revendication 10,
dans lequel les parties de contact avec la peau sont destinées à être ajustées entre les lèvres d'un organe génital féminin lorsque l'article hygiénique absorbant est porté.

19. Article hygiénique absorbant selon la revendication 18,
qui peut être retenu sur le corps d'une personne porteuse par la force de fermeture des lèvres, sans disposer sur la face externe de la couche arrière aucun adhésif sensible à la pression pour empêcher une dislocation de l'article en cours d'utilisation.

20. Article hygiénique absorbant selon la revendication 18,
dans lequel la longueur des parties tridimensionnelles est de 50 à 70 mm dans la direction longitudinale de l'article, et leur largeur entre leurs extrémités libres est de 20 à 40 mm dans une direction perpendiculaire à la direction longitudinale.

21. Article hygiénique absorbant selon la revendication 18,
dans lequel la longueur du corps principal est de 80 à 120 mm dans la direction longitudinale de l'article, et sa largeur est de 40 à 60 mm dans une direction perpendiculaire à la direction longitudinale.

22. Article hygiénique absorbant selon la revendication 10,
dans lequel le corps principal comprend en outre une couche superficielle perméable aux liquides recouvrant la couche absorbante, et les parties tridimensionnelles sont fixées par leurs extrémités fixes sur la couche superficielle.
